# EUROPEAN PATENT SPECIFICATION

(11) **EP 2 689 758 B1**
(45) Date of publication and mention of the grant of the patent: **17.01.2018**
(21) Application number: 12760209.2
(22) Date of filing: 20.02.2012
(51) Int. Cl.: A61F 13/15, A61F 13/49, B65G 47/68

(54) **ABSORBENT ARTICLE PRODUCTION METHOD**
VERFAHREN ZUR HERSTELLUNG EINES SAUGFÄHIGEN ARTIKELS
PROCÉDÉ DE PRODUCTION D'ARTICLE ABSORBANT

(30) Priority: 24.03.2011 JP 2011066455
(43) Date of publication of application: 29.01.2014
(73) Proprietor: Unicharm Corporation, Ehime 799-0111 (JP)
(72) Inventor: MURAKAMI, Seiji, Kanonji-shi Kagawa 769-1602 (JP)
(74) Representative: Peter, Julian
(86) International application number: PCT/JP2012/053916
(87) International publication number: WO 2012/127954

(56) References cited:
- JP-A- 54 088 553
- JP-A- 2003 038 566
- JP-A- 2003 508 243
- JP-A- 2004 168 449
- JP-A- 2007 125 407
- JP-A- 2010 195 534
- JP-A- 2010 527 668

## Description

### [Technical Field]

The present invention relates to a method for manufacturing an absorbent article such as a pant-type diaper.

### [Background Art]

A method for manufacturing an absorbent article such as a pant-type diaper, for example, includes a step of conveying a web that configures a component of the absorbent article in a continuous state, a step of mounting an elastic member and an absorber on the web that is conveyed, a folding step of folding the web along the conveyance direction, a step of joining the web at a position that forms both the waist side portions of the absorbent article, and a step of forming individual absorbent articles by cutting the joint portion (for example, see Patent Document 1). Each absorbent article is in a state such that both the waist side portions of the absorbent article are adjacent to each other, that is, both the waist side portions of the absorbent article arranged side by side.

Because individual absorbent articles are formed by cutting the continuous web, an insufficient interval is provided between the absorbent articles when the absorbent article is cut into individual units, which could lead to interference between the absorbent articles during conveyance. In order to prevent such interference between the absorbent articles, the conveyance path of the absorbent article is switched to a conveyance path with a fast speed, for example, so as to widen the interval between the absorbent articles.

Furthermore, generally, the absorbent articles are counted by a counting machine, and a predetermined number of absorbent articles are stored in a box-shaped package, for example. At this time, as the absorbent articles are arranged side by side, an absorbent article is loaded in to the counting machine from the side of the waist side portion. As the waist side portions of the absorbent article have a lower rigidity than a crotch portion in which the absorber is provided, the waist side portions get deformed easily. When the absorbent article is loaded in to the counting machine in a deformed state, a part of the absorbent article might get caught in the counting machine, and depending on the counting machine, it might not be possible to smoothly feed the absorbent articles in to the counting machine.

In view of such a problem, after the absorbent article is cut, the direction of the absorbent article is changed (for example, see Patent Document 2). In Patent Document 2, a technology of arranging the absorbent articles on a plurality of mounting blocks provided on the circumference of a rotating table, and then rotating the absorbent articles by 90 degrees in a direction crossing the conveyance direction as a result of the rotation of the rotating table so as to change the direction of the absorbent articles is disclosed.

### [Prior Art Document]

### [Patent Document]

Patent Document 1: Japanese Unexamined Patent Application Publication No. 2003-38566 (Fig. 1 etc.)
Patent Document 2: Japanese Unexamined Patent Application Publication No. 2004-168449 (Fig. 1 etc.)
WO2008/141834 A1 discloses a method for producing a diaper whereby the main web constituting the absorbent diaper is separated in two parts in the machine direction.

### [Summary of Invention]

However, as described above, if the direction of the absorbent articles is changed by using the rotating table when the conveyance speed of the absorbent articles is increased to prevent interference between the absorbent articles, the rotating speed of the rotating table increases, and the centrifugal force exerted on the absorbent articles arranged on the mounting block increases. Thus, when the direction of the absorbent articles is changed, the absorbent articles might fall from the mounting block due to the centrifugal force, or the position of the absorbent articles might shift due to the centrifugal force.

Thus, the present invention has been made in view of such a situation and its purpose is to provide a method for manufacturing an absorbent article that can prevent the occurrence of problems during a change of direction of the absorbent article while preventing interference between the absorbent articles.

A method for manufacturing the absorbent article according to the present disclosure is summarized by camprising: a mounting step (component mounting step S1) of conveying a web (web 7) in which components forming the absorbent article, having a front waistline region, a back waistline region, and a crotch region positioned between the front waistline region and the back waistline region, are sequentially arranged, and then mounting an absorber on the web; a folding step (folding step S3) of folding the web along the conveyance direction, a cutting step (cutting step S5) of cutting the web and forming a plurality of absorbent articles, an assigning step (assigning step S6) of assigning the absorbent articles cut by the cutting step to at least a first path and a second path, one by one; and a direction changing step (direction-changing step S8) of changing the direction of the absorbent articles in at least one of the paths of the first path and the second path, wherein the absorbent article has a front waistline region (front waistline region 10), a back waistline region (back waistline region 20), and a crotch region (crotch region 30) positioned between the front waistline region and the back waistline region.

### [Brief Description of Drawings]

[Fig. 1] Fig. 1 is a perspective view showing an absorbent article according to the present embodiment.
[Fig. 2] Fig. 2 is a diagram for explaining a part of the method for manufacturing the absorbent article according to the present embodiment.
[Fig. 3] Fig. 3 is a diagram for explaining a part of the method for manufacturing the absorbent article according to the present embodiment.
[Fig. 4] Fig. 4 is a diagram for showing a schematic view of the conveyance status of the absorbent article.

### [Description of Embodiments]

Hereinafter, the method for manufacturing the absorbent article according to the present embodiment is explained with reference to drawings. Specifically, (1) Configuration of the absorbent article, (2) Method for manufacturing the absorbent article, (3) Aspect of conveyance of the absorbent article, and (4) Other embodiments are explained.

In the following description of the drawings, the same or similar reference numerals are used to designate the same or similar parts. It should be noted that the diagrams are schematic and ratios of the respective dimensions do not determine the actual ones. Therefore, the specific dimensions, etc., should be determined in consideration of the following explanations. Moreover, it is needless to say that relations and ratios among the respective dimensions may differ among the diagrams.

### (1) Configuration of the absorbent article

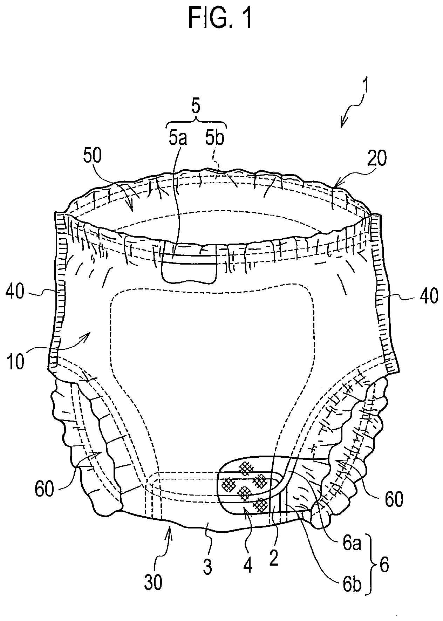

A configuration of an absorbent article according to the present embodiment is explained with reference to drawings. Fig. 1 is a perspective view showing the absorbent article according to the present embodiment. In the present embodiment, an absorbent article 1 is a disposable diaper for adults.

As shown in Fig. 1, roughly, the absorbent article 1 is configured by a liquid-permeable topsheet 2 that is in contact with the skin of the wearing subject (hereinafter, the wearer), a backsheet 3 provided on the outer side from the topsheet 2, and an absorber 4 that is provided between the topsheet 2 and the backsheet 3 and absorbs excrement from the wearer.

Note that a liquid-impermeable water-resistive sheet (not shown in the figure) is provided between the backsheet 3 and the absorber 4. In other words, the absorber 4 is provided between the topsheet 2 and the water-resistive sheet.

A nonwoven fabric or an aperture plastic film is used for the topsheet 2. A nonwoven fabric is used for the backsheet 3. Ground pulp or a mixture of ground pulp and high absorbent polymer grains is used for the absorber 4. A plastic film, a nonwoven fabric, or a combined sheet of a plastic film and a nonwoven fabric is used for the water-resistive sheet.

The absorbent article 1 has a front waistline region 10 applied to the front waistline of the wearer, a back waistline region 20 applied to the back waistline of the wearer, and a crotch region 30 applied to the crotch of the wearer.

The front waistline region 10 and the back waistline region 20 are jointed at the joint unit 40. Waist gathers 5 are configured by thread-shaped rubber having elasticity are provided on the circumference of the front waistline region 10 and the back waistline region 20. The waist gathers 5 are configured by a front waist gathers 5a positioned in the front waistline region 10 and a back waist gathers 5b positioned in the back waistline region 20. A waistline aperture region 50 is formed between the front waist gathers 5a and the back waist gathers 5b.

The crotch region 30 is provided between the front waistline region 10 and the back waistline region 20. Leg gathers 6 configured by thread-shaped or band-shaped rubber having elasticity are provided on both sides of the crotch region 30. The leg gathers 6 are configured by a front leg gathers 6a positioned towards the front waistline region 10 and a back leg gathers 6b positioned towards the back waistline region 20. A leg- surrounding opening region 60 is formed between the front leg gathers 6a and the back leg gathers 6b, and on both sides of the crotch region 30.

### (2) Method for manufacturing the absorbent article.

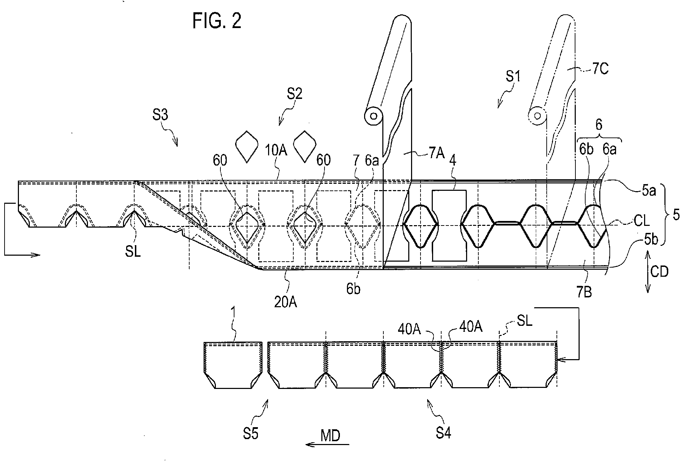

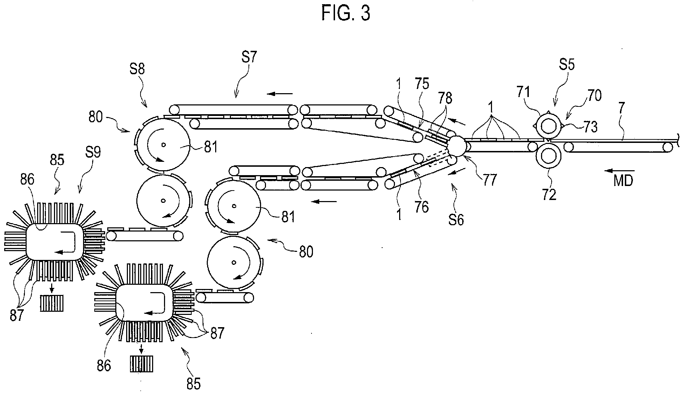

Next, a method for manufacturing the absorbent article according to the present embodiment is explained with reference to drawings. Fig. 2 and Fig. 3 are diagrams for explaining a part of the method for manufacturing the absorbent article according to the present embodiment. Fig. 2 is a diagram for explaining the component mounting step, the leg-surrounding opening forming step, the folding step, the joining step, and the cutting step of the method for manufacturing the absorbent article, and is a schematic view when the step of manufacturing the absorbent article is seen from the top. Fig. 3 is a diagram for explaining the cutting step, the assigning step, the reducing speed step, and the direction-changing step of the method for manufacturing the absorbent article, and is a schematic view when the step of manufacturing the absorbent article is seen from the side.

As shown in Fig. 2 and Fig. 3, the method for manufacturing the absorbent article includes at least the component mounting step, the leg-surrounding opening forming step, the folding step, the joining step, the cutting step, the assigning step, the reducing speed step, the direction-changing step, and the counting step. Note that a step of conveying a liquid-permeable first web 7A configuring the topsheet 2, a liquid-impermeable second web 7B configuring the backsheet 3, and a third web 7C made from the same material as the second web 7B and configuring the backsheet 3 in the conveyance direction MD using a conveyor (for example, a belt conveyor), which is not shown in the figure, is included between the component mounting step, the leg-surrounding opening forming step, the folding step, the joining step, and the cutting step.

### (2-1) Component mounting step

In the component mounting step S1, the components configuring the absorbent article 1, such as the elastic members, the third web 7C, the water-resistive sheet (not shown in the figure), and the absorber 4 are mounted on the second web 7B.

### (2-2) Leg-surrounding opening forming step

In the leg-surrounding opening forming step S2, after the component mounting step S1, the leg- surrounding opening region 60 (the so-called leg hole) is formed on the second web 7B and the first web 7A (hereinafter, web 7) between which the components are held, by cutting out both sides of the crotch region 30.

### (2-3) Folding step

In the folding step S3, after the leg-surrounding opening forming step S2, a web 7 is folded into two through the center of the direction CD crossing the web 7 and along the central line CL facing the conveyance direction MD. In other words, a side edge 10A of the web 7 corresponding to the front waistline region 10, and a side edge 20A of the web 7 corresponding to the back waistline region 20 overlap in a matching state.

### (2-4) Joining step

In the joining step S4, after the folding step S3, a predetermined region 40A corresponding to the joint unit 40 of the absorbent article 1 is joined with an ultrasonic treatment and a heat treatment. Note that the predetermined region 40A shows both sides of the conveyance direction MD of the virtual line SL showing the estimated cutting position extending in the cross direction CD.

### (2-5) Cutting step

In the cutting step S5, after the joining step S4, the web 7 with which the predetermined region 40A is joined is cut along the virtual line SL by a cutting device 70 shown in Fig. 3. The absorbent article 1 is thus formed. The cutting device 70 has a cutter roller 71 and an anvil roller 72 which faces cutter roller 71. A cutting blade 73 configured to cut the web 7 is provided on the circumference surface of the cutter roller 71. The web 7 is cut by the cutting blade 73 at a fixed interval when the web 7 passes through between the cutter roller 71 and the anvil roller 72.

### (2-6) Assigning step

In the assigning step S6, the absorbent articles 1 cut by the cutting step S5 are assigned to at least a first path 75 and a second path 76, one by one. Note that the conveyance speed of the first path 75 and the second path 76 assigned by the assigning step is almost the same as the conveyance speed of the absorbent article prior to assigning. Thus, a gap equivalent to one absorbent article is formed between the adjoining absorbent articles 1 assigned to the first path 75 and the second path 76. An assigning device 77 has a guide member 78 that feeds the absorbent articles. The guide member 78 is configured to swing with the axis of rotation as the center, and due to the swing, the absorbent articles 1 are supplied alternately to the first path 75 and the second path 76.

### (2-7) Reducing speed step

In the reducing speed step S7, the absorbent articles 1 conveyed in the first path 75 are switched to a path with a conveyance speed slower than the conveyance speed of the assigning step S6 so as to slow down the conveyance speed of the absorbent articles, and the absorbent articles 1 conveyed in the second path 76 are switched to a path with a conveyance speed slower than the conveyance speed of the assigning step so as to slow down the conveyance speed of the absorbent articles. By reducing the conveyance speed by the reducing speed step S7, the gap between the conveyed absorbent articles 1 can be adjusted.

### (2-8) Direction-changing step

In the direction-changing step S8, the direction of the absorbent articles 1 conveyed in the first path 75 and the direction of the absorbent articles 1 conveyed in the second path 76 are changed by a direction-changing device 80. The direction-changing device 41 includes a rotating drum 81 and a plurality of adsorption units (not shown in the figure) provided on the circumference surface of the rotating drum 81. The adsorption units are configured so as to adsorb the absorbent articles 1, and rotate the absorbent articles 1 by 90 degrees with respect to the circumference surface of the rotating drum 81 while moving along the circumference surface of the rotating drum 81. The direction of the absorbent articles is thus changed.

Note that by providing the reducing speed step S7, the processing efficiency in the direction-changing step S8 can be improved. For example, if the interval between the absorbent articles 1 being conveyed towards the direction-changing step S8 becomes too much, the number of absorbent articles that can be arranged in the adsorption units of the rotating drum becomes less, which might cause the processing efficiency to decline. However, by including the reducing speed step S7, the interval between the absorbent articles can be shortened thereby increasing the processing efficiency of the direction-changing step S8.

### (2-9) Counting step

In the counting step S9, the absorbent articles whose direction has been changed by the direction-changing step S8 are counted by the counting machine 85. The counting machine 85 has an endless chain 86 that moves in a fixed direction, and a plurality of guide plates 87 provided on the endless chain 86 at equal intervals. The absorbent articles are configured to be held between the plurality of guide plates 87. The absorbent articles 1 are sandwiched by the guide plates 87 and are conveyed in an aligned state. The counting machine 85 has a pusher (not shown in the figure) that pushes the absorbent articles 1 conveyed in the aligned state in the lower direction. Thus, the absorbent articles are pushed out in predetermined quantities. Note that although not shown in the figure, after the counting step S9, the absorbent articles pushed out in predetermined quantities are stored in a box-shaped package in predetermined quantities of the absorbent article.

### (3) Aspect of conveyance of the absorbent article

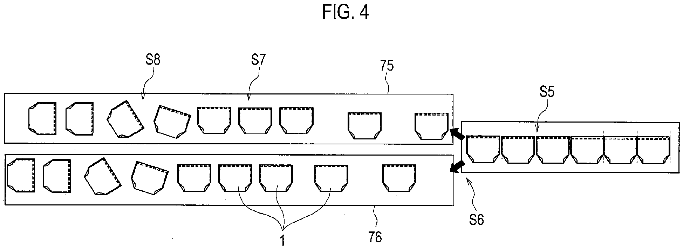

Next, the form of conveyance of the absorbent article thus manufactured is explained based on Fig. 4. Fig. 4 is a diagram showing a schematic view of the conveyance status of the absorbent article after the cutting step. Note that in the explanation in Fig. 4, the conveyance speed is explained as being fixed.

As for the absorbent articles 1 cut in the cutting step S5, the gap between the adjoining absorbent articles is not sufficient; this causes the absorbent articles to come in close vicinity of each other. Due to the assignment of the absorbent articles 1 to the first path 75 and the second path 76 in the assigning step S6, the interval between the absorbent articles becomes equivalent to a single absorbent article 1. In the reducing speed step S7, by switching the conveyance speed of the absorbent article to a low speed, the interval between the absorbent articles is shortened.

The absorbent articles 1 whose interval has been shortened in the reducing speed step S7 are rotated by 90 degrees in the direction-changing step S8, and change from the horizontally-arranged state to the vertically-arranged state. The absorbent articles 1 that have thus been changed to the vertically-arranged state are loaded in to the counting machine from the crotch region S30 side. Thus, the absorbent articles 1 can be loaded smoothly in to the counting machine 85.

As described above, according to the present embodiment, because the absorbent articles are assigned one by one to a plurality of paths after cutting the web, an interval can be provided between the absorbent articles without increasing the conveyance speed of the absorbent articles. Thus, interference between the absorbent articles can be prevented. Because an interval can be provided between the absorbent articles without increasing the conveyance speed of the absorbent articles, the occurrence of problems during the change of direction of the absorbent articles can be prevented.

### (4) Other embodiments

Needless to say, the present invention includes various embodiments not described herein. Therefore, the technical range of the present invention is to be defined only by the inventive specific matter according to the adequate claims from the above description.

For example, the present embodiment is configured such that the absorbent articles are assigned to the first path and the second path in the assigning step, however, the present invention is not limited to this configuration, and, for example, the absorbent articles can be assigned to three or more paths.

Furthermore, the present embodiment is configured such that the interval between the absorbent articles is adjusted in the deceleration step after the assigning step, but the reducing speed step may not necessarily be included. The present embodiment is configured such that the absorbent articles are counted in the counting step after the direction changing step, but the counting step may not necessarily be included.

The present embodiment is configured such that the direction of the absorbent articles conveyed in the first path as well as that of the absorbent articles conveyed in the second path is changed, however, the configuration may be such that the direction of the absorbent articles conveyed in at least either one of the paths can also be changed.

### [Industrial Applicability]

According to the present embodiment, because the absorbent articles are assigned one by one to a plurality of paths after cutting the web, an interval can be provided between the absorbent articles without increasing the conveyance speed, of the absorbent articles. Thus, interference between the absorbent articles can be presented. Because an interval can be provided between the absorbent articles without increasing the conveyance speed of the absorbent articles, a method for manufacturing an absorbent article that can prevent the occurrence of problems during the change of direction of the absorbent articles can be provided.

### [Explanation of Numerals]

- 1: : absorbent article
- 2: : topsheet
- 3: : backsheet
- 4: : absorber
- 5: : waist gathers
- 5a: : front waist gathers
- 5b: : back waist gathers
- 6: : leg gathers
- 6a: : front leg gathers
- 6b: : back leg gathers
- 7: : web
- 7A: : first web
- 7B: : second web
- 7C: : third web
- 10: : front waistline region
- 10A: : side edge
- 20: : back waistline region
- 20A: : side edge
- 30: : crotch region
- 40: : joint unit
- 40A: : predetermined region
- 50: : waistline aperture region
- 60: : leg- surrounding opening region
- 70: : cutting device
- 71: : cutter roller
- 72: : anvil roller
- 73: : cutting blade
- 75: : first path
- 76: : second path
- 77: : assigning device
- 78: : guide member
- 80: : direction-changing device
- 81: rotating drum
- 85: : counting machine
- 86: : endless chain
- 87: : guide plates

## Claims

1. A method for manufacturing an absorbent article (1), comprising:
a mounting step of conveying a web (7) in which components forming the absorbent article (1), having a front waistline region (10), a back waistline region (20), and a crotch region (30) positioned between the front waistline region (10) and the back waistline region (20), are sequentially arranged, and then mounting an absorber (4) on a web (7B) of the web (7);
a folding step of folding the web (7) along the conveyance direction;
a cutting step of cutting the web (7) and forming a plurality of absorbent articles (1);
an assigning step of assigning the absorbent articles (1) cut by the cutting step to at least a first path (75) and a second path (76), one by one;
after the assigning step, a reducing speed step of reducing the conveyance speed of the absorbent articles (1) by switching to a path with a conveyance speed slower than the conveyance speed of the assigning step, and
a direction-changing step of changing the direction of the absorbent articles (1) in at least one of the paths of the first path (75) and the second path (76).

2. The method for manufacturing an absorbent article according to claim 1, further comprising, after the direction-changing step, a counting step of counting the absorbent articles (1) by a counting machine.

## Patentansprüche

1. Eine Methode einen absorbierenden Gegenstand (1) herzustellen, umfassend:
einen Montageschritt des Förderns eines Gewebes (7), wobei Komponenten, die den absorbierenden Gegenstand (1), mit einem vorderen Taillenbereich (10), einem hinteren Taillenbereich (20), einem Schrittbereich (30), der zwischen dem vorderen Taillenbereich (10) und dem hinteren Taillenbereich (30) positioniert ist, ausbilden, nacheinander angeordnet sind, und dann einen Absorber auf einem Gewebe (7B) des Gewebe (7) anbringen;
einen Faltschritt des Faltens des Gewebes (7) entlang der Förderrichtung;
einen Schneideschritt des Schneidens des Gewebes (7) und eine Vielzahl an absorbierenden Gegenständen (1) ausbildend;
einen Zuordnungsschritt des Zuordnens der absorbierenden Gegenstände (1), die durch den Schneideschritt geschnitten wurden, zu mindestens einem ersten Pfad (75) und einem zweiten Pfad (76), einer nach dem anderen;
nach dem Zuordnungsschritt, einen Geschwindigkeitsverzögerungsschritt des Verzögerns der Fördergeschwindigkeit der absorbierenden Gegenstände (1) durch Wechsel zu einem Pfad mit einer langsameren Fördergeschwindigkeit als die Fördergeschwindigkeit des Zuordnungsschrittes, und
einem Richtungswechselschritt des Richtungswechselns der absorbierenden Gegenstände (1) bei mindestens einem der Pfade des ersten Pfads (75) und des zweiten Pfads (76).

2. Die Methode einen absorbierenden Gegenstand herzustellen gemäß Anspruch 1, weiter umfassend, nach dem Richtungswechselschritt, einen Zählschritt des Zählens der absorbierenden Gegenstände (1) durch eine Zählmaschine.

## Revendications

1. Procédé de fabrication d'un article absorbant (1) comprenant:
une étape de transportation du tissu (7), dans lequel les components composants l'article absorbant (1) ayant une région de taille avant (10), une région de taille arrière (20), une région d'entrejambe (30) disposée entre la région de taille avant (10) et la région de ceinture arrière (30) sont positionnée agencée l'une après l'autre, et ensuite attachant un absorbeur à un tissu (7B) du tissu (7);
une étape de pliage consistant à plier le tissu (7) le long de la direction de transport;
une étape de coupe consistant à couper le tissu (7) et à former une pluralité d'articles absorbants (1);
une étape d'association consistant à associer les articles absorbants (1) coupés par l'étape de coupe avec au moins un premier trajet (75) et un second trajet (76) l'un après l'autre;
après l'étape d'allocation, une étape de retardement de la vitesse de retardement de la vitesse de transport des articles absorbants (1) en passant à un trajet ayant une vitesse de transport plus lente que la vitesse de transport de l'étape d'association, et
une étape de changement de direction consistant à inverser la direction des articles absorbants (1) au niveau d'au moins l'un des trajets du premier trajet (75) et du second trajet (76).

2. Procédé de fabrication d'un article absorbant selon la revendication 1, comprenant en outre, après l'étape de changement de direction, une étape de comptage consistant à compter les articles absorbantes (1) à l'aide d'une machine à compter.
